(19) Europäisches Patentamt
European Patent Office
Office européen des brevets

(11) Publication number: 0 028 810 A2

# (12) EUROPEAN PATENT APPLICATION

(21) Application number: 80106826.3

(22) Date of filing: 06.11.80

(51) Int. Cl.³: C 08 G 59/06

(30) Priority: 08.11.79 US 92410

(43) Date of publication of application: 20.05.81 Bulletin 81/20

(84) Designated Contracting States: CH DE FR GB IT LI NL

(71) Applicant: THE DOW CHEMICAL COMPANY
2030 Abbott Road Post Office Box 1967
Midland, Michigan 48640(US)

(72) Inventor: Massingill, John Lee
410 Forest Drive
Lake Jackson Texas(US)

(74) Representative: Hann, Michael, Dr. et al,
Postfach 5524
D-6300 Giessen 1(DE)

## (54) Solvent-free process for preparing liquid epoxy resins.

(57) Liquid epoxy resins are produced in solvent-free process by (1) coupling a dihydric phenol with an excess of an epihalohydrin in the presence of a suitable catalyst, (2) removing the excess epihalohydrin, (3) dehydrohalogenating the resultant solvent-free halohydrin intermediate at an elevated temperature and (4) removing inorganic reaction product materials by water washing the resultant solvent-free liquid epoxy resin at elevated temperature to obtain a liquid epoxy resin.

EP 0 028 810 A2

# SOLVENT-FREE PROCESS FOR PREPARING LIQUID EPOXY RESINS

The known processes for producing epoxy resins employ a solvent during dehydrohalogenation and/or during washing. Some processes, K. B. Cofer in U.S. 3,413,320 and H. P. Price et al in U.S. 3,221,032, employ excess epihalohydrin as the solvent for epoxidation and washing. Other processes, A. G. Farnham et al in U.S. 2,943,095, N. H. Reinking in U.S. 2,943,096, L. H. Griffin et al in U.S. 2,986,551 and S. Vargin in U.S. 4,017,523, employ toluene and/or other solvents during epoxidation and washing. Still other processes, H. P. Price et al in U.S. 3,352,825 and T. J. Hairston et al in U.S. 3,767,618 employ no solvent during epoxidation but employ such solvents as ketones during the washing step.

The use of solvents in resin processing is disadvantageous because the presence of extraneous substances usually causes a decrease in the purity of

the product. Reactive solvents can give rise to secondary reactions with formation of undesirable by-products. In each instance, there is a necessity for separating the added solvent from the liquid resin and from the waste water streams from the washing step as well as the necessity to purify the solvents for recycle purposes.

The difficulty and lack of spontaniety in the separation of liquid epoxy resins from water or aqueous saline solutions is well known as described by S. Vargin et al in U.S. 4,017,523. In order to facilitate this separation, substances capable of varying the interfacial tension or the density have been employed. These extraneous substances decrease the purity of the resin and must be removed which often is difficult to accomplish. When operating without these extraneous substances, lengthy periods of decantation at elevated temperatures are required which gives rise to undesirable secondary reactions.

It has now been discovered that it is possible to eliminate the use of solvents in the epoxidation and washing steps in the production of liquid epoxy resins. This essentially eliminates or substantially reduces the disadvantages encountered in the known processes for preparing liquid epoxy resins. The process of the present invention provides for the production of liquid glycidyl ethers of polyhydric phenols in high purity, with low total chloride, substantially free of hydrolyzable chloride and with virtually no ionic chloride.

The present invention provides a process for preparing liquid diglycidyl ethers of dihydric phenols which process comprises

(A) reacting an epihalohydrin with a dihydric phenol in a mole ratio of from 4:1 to 20:1, preferably from 5:1 to 15:1 and most preferably from 6:1 to 10:1 at a temperature of from 25°C to 150°C, preferably from 30°C to 100°C and most preferably from 42°C to 75°C in the presence of a catalytic quantity of a suitable catalyst for the coupling reaction for a time sufficient to provide a residual phenolic hydroxyl content of less than about 0.5 percent by weight of the reaction mixture;

(B) removing the excess epihalohydrin and by-products by means of thin film distillation at a temperature of no higher than 130°C thereby obtaining an intermediate halohydrin-containing product;

(C) dehydrohalogenating the intermediate product with from about 5 to about 70 percent by weight, preferably from about 10 to about 30 percent by weight, most preferably from about 18 to about 27 percent by weight aqueous alkali metal hydroxide solution in a quantity to provide from about 100 to about 500 percent, preferably from about 100 to about 300 percent and most preferably from about 100 to about 200 percent of that theoretical quantity required to dehydrohalogenate all of the

halohydrin groups contained in the intermediate product at a temperature of from 50°C to 105°C, preferably from 60°C to 100°C, most preferably from 65°C to 95°C; and

(D) washing the resultant dehydrohalogenated product with water at a temperature of from 50°C to 100°C characterized by dehydrohalogenating the intermediate product in step (C) and washing the resultant dehydrohalogenated product in step (D) in the absence of an organic solvent.

The epoxy resins, essentially monomeric, diglycidyl ethers of dihydric phenols, prepared by the process of the present invention have a total halide content of less than 0.5 percent, hydrolyzable halide content of less than 0.05 percent and an ionic halide content of less than 10 ppm; preferably, the total halide content is less than 0.4 percent, the hydrolyzable halide content is less than 0.03 percent and the ionic halide content is less than 5 ppm; and most preferably, the total halide content is less than 0.3 percent, the hydrolyzable halide content is less than 0.02 percent and the ionic halide content is less than 2 ppm.

Suitable dihydric phenols which can be employed herein include, for example, hydroquinone, resorcinol, catechol, and bisphenols such as those represented by the formula

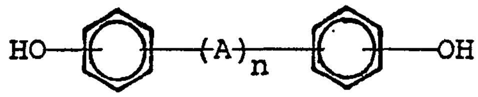

wherein A is a divalent hydrocarbon group having from 1 to about 10 carbon atoms, -S-, -S-S-, $-\overset{O}{\overset{\|}{S}}-$, $-\underset{O}{\underset{\|}{\overset{O}{\overset{\|}{S}}}}-$ or $-\overset{O}{\overset{\|}{C}}-$ and n has a value of zero or 1 and mixtures thereof.

Suitable epihalohydrins which can be employed herein include, for example, epichlorohydrin, epiiodohydrin, epibromohydrin and mixtures thereof.

Suitable alkali metal hydroxides which can be employed herein as dehydrohalogenation agents include, for example, sodium hydroxide, potassium hydroxide, lithium hydroxide and mixtures thereof.

Suitable catalysts for the epoxy-phenolic hydroxyl coupling reaction include, for example, quaternary ammonium compounds, quaternary phosphonium compounds, sulfonium compounds, and inorganic bases.

Suitable quaternary ammonium catalysts include, for example, tetramethyl ammonium chloride, benzyl trimethyl ammonium chloride, triethanol ammonium chloride, tetraethanol ammonium hydroxide, and dodecyl dimethylbenzyl ammonium naphthenate.

Suitable quaternary phosphonium catalysts include, for example, those quaternary phosphonium

compounds disclosed in U.S. Patents 3,948,855, U.S. 3,477,990 and U.S. 3,341,580 and Canadian 858,648. Particularly suitable such catalysts include, for example, ethyl triphenyl phosphonium iodide, ethyl triphenyl phosphonium bicarbonate, ethyl triphenyl phosphonium acetate·acetic acid complex, benzyl triphenyl phosphonium chloride, tetrabutyl phosphonium chloride, benzyl trimethyl ammonium chloride and mixtures thereof.

Suitable sulfonium catalysts include thiourea catalysts such as, for example, tetramethyl thiourea; N,N'-dimethyl thiourea; N,N'-diphenyl thiourea; and mixtures thereof as well as thiodiethanol and other sulfonium precursors.

Suitable inorganic base catalysts for coupling include, for example, sodium hydroxide, sodium carbonate, trisodium phosphate, sodium silicate, basic ion exchange resins, and mixtures thereof.

The residual dihydric phenol content of the coupling reaction, step A, can be determined by colorimetrically using Mellon's reagent, spectrophotometrically using an ultraviolet spectrophotometer or chromotographically using a liquid chromotograph or a gas chromotograph.

The thin film distillation can be performed by any of the known methods such as, for example, by the use of falling film stills, wiping film stills, and centrifugal stills.

The dehydrohalogenation step (B) can be conveniently conducted in most any type of vessel; however, particularly suitable are mixer-settlers, reciprocating plate columns, and mass transfer apparatus.

Reciprocating plate columns are commercially available from Chem-Pro Equipment Corporation.

Mass transfer apparatus are commercially available from Merichem Company as FIBER-FILM contactors and are described in U.S. Patents 3,758,404; 3,992,156 and 3,977,829.

EXAMPLE 1

Step (A) Coupling

A mixture of epichlorohydrin and bisphenol A in a 10:1 molar ratio was mixed with an amount of benzyltrimethyl ammonium chloride to a level of 0.24 weight percent. This mixture was coupled in a continuous stirred tank reactor system consisting of 5 reactors at 43°C, 43°C, 65°C, 70°C and 70°C, respectively. Residence time in each reactor was one day. The final coupled mix contained 0.18 weight percent residual bisphenolics as determined by ultraviolet spectroscopy. Chromotographic analysis of the mixture gave the following composition:

| | Weight Percent |
|---|---|
| epichlorohydrin | 53.3 |
| glycerin dichlorohydrin | 12.6 |
| diglycidyl ether bisphenol A (DGEBA) | 10.5 |
| monochlorohydrin of DGEBA | 13.3 |
| dichlorohydrin of DGEBA | 5.0 |
| resin, $n \geq 1$ | 3.9 |
| miscellaneous | 1.5 |

Step (B) Epichlorohydrin Removal

The coupled solution of Step (A) above was distilled in a 0.25 $ft^2$ (232 $cm^2$) glass wiping film still equipped with teflon wipers, heating mantle, thermocouple, pyrometer, temperature controller, dry ice condensor, vacuum manometer and vacuum pump. The coupled solution was fed to the still at 20 cc/minute. The still was operated at a temperature of 130°C and a vacuum of 30 mm Hg. The neat resin intermediate produced contained 0.16 percent epichlorohydrin, 0.28 percent glycerine dichlorohydrin, 7.1 percent hydrolyzable chloride and 7800 ppm benzyltrimethyl ammonium chloride.

Step (C) Dehydrohalogenation

200 grams of the neat resin intermediate of Step (B) above was heated with stirring to 75°C in a two liter flask equipped with a bottom valve, thermometer, stirrer and reflux condensor. Then 75 grams (12 percent excess) of 25 weight percent sodium hydroxide aqueous solution was added. Stirring was continued for 32 minutes and the aqueous phase removed to leave the neat dehydrohalogenated crude resin.

Step (D) Washing

To the dehydrohalogenated neat resin of Step (C) above was added 100 cc of distilled water at 70°C. The mixture was stirred vigorously and then pumped through a coalescer/separator at 70°C. The washed resin was collected and the procedure repeated once more.

The epoxy resin from Step (D) above was distilled in a wiping film still (described in Step (B) above) at a temperature of 175°C and a vacuum of 0.2 mm Hg. to remove residual lights and water. The resulting resin had the following properties:

| | |
|---|---|
| Epoxide content, weight percent | 23.0 |
| Epoxy equivalent weight | 187 |
| Hydrolyzable chloride, ppm | 210 |
| Ionic chloride, ppm | <1 |
| Total chloride, weight percent | 0.35 |
| Residual catalyst, ppm | 25 |
| Viscosity at 25°C, cps | 11,200 |

<u>EXAMPLE 2</u>

Step (A) and Step (B) were identical to Example 1, Steps (A) and (B).

<u>Step (C) Dehydrohalogenation</u>

Neat resin intermediate of Step (B) was continuously epoxidized in a 1 inch x 5 foot (2.54 cm x 152 cm) glass reciprocating plate column operated at 75°C and 750 strokes per minute. The plate stack contained 33 teflon plates with 50 percent void area on a 2 inch (5 cm) spacing. Stroke length was 1/4 inch (5 cm). Neat resin intermediate (10 cc/min) was fed continuously through the bottom inlet and aqueous 25 weight percent sodium hydroxide (5 cc/min) was fed continuously through the top inlet. Dehydrohalogenated resin overflowed through the top outlet and waste caustic solution was discharged by pump from the bottom outlet.

Step (D) Washing

The dehydrohalogenated resin (5 parts by volume) of Step (C) above was fed continuously into a 70°C, two-stage water-wash/coalesce system where it was twice washed with distilled water (1 part by volume), coalesced and separated.

The resin was finished as described in Example 1. The product resin had the following properties:

| | |
|---|---|
| Epoxide content, weight percent | 23.11 |
| Epoxy equivalent weight | 186 |
| Hydrolyzable chloride, ppm | 200 |
| Ionic chloride, ppm | <1 |
| Total chloride, weight percent | 0.34 |
| Residual catalyst, ppm | 25 |
| Viscosity at 25°C, cps | 11,300 |

EXAMPLE 3

Step (A) Coupling

A mixture of epichlorohydrin and bisphenol A in a 10:1 molar ratio was mixed with an amount of benzyltrimethyl ammonium chloride to a level of 0.24 weight percent in a 2-liter, three neck, round bottom flask equipped with a thermometer, stirrer and reflux condensor. The resulting solution was heated, with stirring, to 45°C and maintained at that temperature for 95 hours to give a solution of coupled resin intermediate in epichlorohydrin containing 0.2 weight percent residual bisphenolics as determined by ultraviolet spectroscopy.

Step (B) Epichlorohydrin Removal

Step (B) is identical to Example 1, Step (B).

Step (C) Dehydrohalogenation

200 grams of the neat resin intermediate of Step (B) were heated with stirring to 74°C in the apparatus described in Example 1, Step (C). Then the stoichiometric amount of caustic carbonate solution (16 percent NaOH, 9 percent $Na_2CO_3$) was added. Stirring was maintained at 1520 rpm for one minute, the stirrer was stopped, the mixture allowed to settle into two phases, and the bottom, aqueous, phase was withdrawn. This procedure was repeated twice more.

Step (D) was identical to Example 1, Step (D). The product resin had the following properties:

| | |
|---|---|
| Epoxide content, weight percent | 23.5 |
| Epoxy equivalent weight | 183 |
| Hydrolyzable chloride, ppm | 380 |
| Ionic chloride, ppm | <1 |
| Total chloride, weight percent | 0.29 |
| Viscosity at 25°C, cps | 6,800 |

1. A process for preparing liquid glycidyl ethers of dihydric phenols having an average of more than one glycidyl ether group per molecule which process comprises:

(A) reacting
an epihalohydrin with
a dihydric phenol in a mole ratio of from 4:1 to 20:1 at a temperature of from 25°C to 150°C, in the presence of a catalytic quantity of a suitable catalyst for the coupling reaction for a time sufficient to provide a residual dihydric phenol content of less than 0.5 percent by weight of the reaction mixture;

(B) removing the excess epihalohydrin and by--products by means of thin film distillation at a temperature of no higher than 130°C thereby forming an intermediate halohydrin--containing product;

(C) dehydrohalogenating the intermediate product with from 5 to 70 percent by weight, aqueous alkali metal hydroxide solution in a quantity to provide from 100 percent to 500 percent of that theoretical quantity required to dehydrohalogenate all of the halohydrin groups contained in the intermediate product at a temperature of from 50°C to 105°C; and

(D) washing the resultant dehydrohalogenated product with water at a temperature of from 50°C to 100°C characterized by dehydrohalogenating the intermediate product in step (C) and washing the resultant dehydrohalogenated product in step (D) in the absence of organic solvent.